# EUROPÄISCHE PATENTSCHRIFT

(11) **EP 2 567 384 B1**
(45) Veröffentlichungstag und Bekanntmachung des Hinweises auf die Patenterteilung: **02.12.2015**
(21) Anmeldenummer: 11717620.6
(22) Anmeldetag: 02.05.2011
(51) Int. Cl.: A61B 6/00, G21F 1/12, A61B 6/10, G21F 3/00

(54) **Strahlenschutzformkörper und dessen Verwendung**
Molded radiation protection part and use thereof
Corps moulé de protection contre les radiations et utilisation dudit corps moulé de protection contre les radiations

(30) Priorität: 05.05.2010 DE 102010028576
(43) Veröffentlichungstag der Anmeldung: 13.03.2013
(73) Patentinhaber: RÖHR + STOLBERG GmbH, 47809 Krefeld (DE)
(72) Erfinder: KISSENBECK, Christoph, 47802 Krefeld (DE)
(74) Vertreter: RGTH
(86) Internationale Anmeldenummer: PCT/EP2011/056926
(87) Internationale Veröffentlichungsnummer: WO 2011/138260

(56) Entgegenhaltungen:
- EP-A1- 0 367 318
- US-A- 2 928 948
- US-A- 4 619 852
- US-A1- 2008 276 554
- US-B1- 6 649 922

## Beschreibung

### Technisches Gebiet

Die Erfindung betrifft einen Strahlenschutzformkörper und dessen Verwendung.

### Stand der Technik

Der Umgang mit ionisierender Strahlung erfordert Maßnahmen zum Schutz des menschlichen Lebens vor Strahlungsschäden. Derartige Schutzmaßnahmen sind überall dort erforderlich, wo ein menschlicher Kontakt mit hohen Dosen an ionisierender Strahlung zu erwarten ist. Dies ist insbesondere in der Energie- und Medizintechnik der Fall. So sind beispielsweise besondere Schutzmaßnahmen bei der Entsorgung von Kernkraftwerken für das Arbeitspersonal zu treffen.

US-A-3 514 607 offenbart ein plattenförmiges Abschirmmaterial, das aus Blei sowie einem Zusatz von Zinn oder Barium besteht. Derartige Platten sind jedoch korrosionsanfällig und nur schwer zu dekontaminieren.

EP-A-1 288 969 offenbart einen Strahlenschutzformkörper, der eine Platte aus Blei umfasst, die ein- oder beidseitig mit einer Abdeckschicht aus Zinn oder einer zinnhaltigen Legierung versehen ist. Eine derartige Abdeckschicht auf der Bleiplatte ermöglicht eine leichtere Dekontamination der Strahlenschutzformkörper. Ferner wird verhindert, dass das Arbeitspersonal in direkten Kontakt mit der Bleiplatte gelangt.

An Strahlenschutzformkörper, die im medizinischen Bereich, insbesondere im medizinischen Therapie- und Diagnostikbereich verwendet werden sollen, werden jedoch zunehmend höhere Anforderungen gestellt. Insbesondere ist es erforderlich, dass eine Reinigung der Oberfläche nicht mit einem Oberflächenabrieb verbunden ist. Ein solcher Oberflächenabrieb könnte mit einer Lösung von Zinnionen verbunden sein, was, so wird gelegentlich befürchtet, mit der Bildung giftiger zinnorganischer Verbindungen verbunden sein könnte.

Ferner werden im medizinischen Bereich besondere hygienische Anforderungen an Strahlenschutzformkörper gestellt. Überdies sind die

Anforderungen an das optische Erscheinungsbild der Abdeckschicht andere als bei der Entsorgung von Kernkraftwerken.

Aus den Druckschriften US 2008/0276554 A1, US 4 619 852 A, US 6 649 922 B1 und US 2 928 948 A sind Strahlenschutzformkörper bekannt, die eine Kleberschicht verwenden.

### Darstellung der Erfindung: Aufgabe, Lösung, Vorteile

Der Erfindung liegt die Aufgabe zugrunde, einen Strahlenschutzformkörper mit verbesserten Eigenschaften anzugeben. Insbesondere soll ein Strahlenschutzformkörper mit einer abriebfesten Abdeckschicht angegeben werden, die strahlungsresistent ist, einen wirksamen Korrosionsschutz bietet und hohen Anforderungen an Hygiene und optisches Erscheinungsbild gerecht wird. Ferner sollen Verwendungen derartiger Strahlenschutzformkörper angegeben werden.

Diese Aufgabe wird durch die Merkmale der Ansprüche 1 und 11 gelöst. Zweckmäßige Ausgestaltungen der Erfindung ergeben sich aus den Unteransprüchen.

Nach Maßgabe der Erfindung ist ein Strahlenschutzformkörper vorgesehen, wobei der Grundkörper ein plattenförmiger, blechförmiger oder folienförmiger Grundkörper mit quadratischem, rechteckförmigem oder eine andere geometrische Form aufweisendem Zuschnitt ist und wobei der Grundkörper eine erste Flächenseite aufweist, die mit einer ersten Beschichtung versehen ist, die aus einem ersten nicht-metallischen Material besteht.

Die Beschichtung deckt die erste Oberfläche vorzugsweise vollständig ab. Vorzugsweise ist der Grundkörper ein biegsamer Grundkörper.

Die Beschichtung der ersten Flächenseite mit dem ersten nicht-metallischen Material bietet im Vergleich zum Stand der Technik eine Vielzahl von Vorteilen. Zum einen wird eine Korrosion der Bleioberfläche des Grundkörpers verhindert und eine bessere Verschleißfestigkeit im Vergleich zu unbeschichtetem Blei erreicht, zum anderen verleiht die Beschichtung aus dem nicht-metallischen Material dem Strahlenschutzformkörper eine besonders geeignete Oberfläche ohne Abrieb und einen vorteilhaften optischen Eindruck.

Aufgrund dieser Vorteile ist der erfindungsgemäße Strahlenschutzformkörper insbesondere für Verwendungen im medizinischen Bereich, insbesondere im

Therapie- und Diagnostikbereich geeignet. Der erfindungsgemäße Strahlenschutzformkörper verfügt somit über verbesserte hygienische, optische und handhabungstechnische Eigenschaften.

In einer Ausführungsform der Erfindung kann die Beschichtung ganz oder teilweise farbig sein, was eine Kennzeichnung des Strahlenschutzformkörpers nach bestimmten Eigenschaften, beispielsweise der Materialstärke des Grundkörpers oder der zur Bildung der Grundkörpers verwendeten Legierung, ermöglicht.

Der erfindungsgemäße Strahlenschutzformkörper weist eine zweite Flächenseite auf, die mit einer Beschichtung versehen ist, wobei die Beschichtung aus einem zweiten nicht-metallischen Material besteht. Das erste nicht-metallische Material und das zweite nicht-metallische Material können gleich oder unterschiedlich sein, wobei es bevorzugt ist, dass die erste und die zweite Flächenseite mit demselben nicht-metallischen Material beschichtet sind. Die Beschichtung deckt die zweite Flächenseite vorzugsweise vollständig ab.

Die Stärke der Beschichtung aus einem nicht-metallischen Material beträgt 10 nm bis 100 µm, besonders bevorzugt 1 bis 50 µm und am stärksten bevorzugt 10 bis 30 µm. Insbesondere ermöglicht der letztgenannte Bereich eine noch deckende Beschichtung, die sowohl die Anforderung, aus wirtschaftlichen Gründen möglichst dünn ausgebildet zu sein, als auch die Anforderung, eine Dicke aufzuweisen, die Verformungen insbesondere der "Tiefziehen" ermöglicht, erfüllt. Handelt es sich bei dem Grundkörper um einen folienförmigen Grundkörper, so liegt die Stärke der Beschichtung aus einem nicht-metallischen Material stärker bevorzugt zwischen 10 und 500 nm, besonders bevorzugt zwischen 50 und 250 nm.

Ferner kann vorgesehen sein, dass sämtliche Oberflächen, d. h. die beiden Flächenseiten und die Schmalseiten des Grundkörpers, mit einer Beschichtung aus nicht-metallischen Materialien versehen sind, wobei es bevorzugt ist, dass die Schmalseiten mit demselben nicht-metallischen Material wie die erste und/oder die zweite Flächenseite beschichtet sind.

Gemäß der Erfindung weist der Strahlenschutzformkörper zusätzlich zu einer Beschichtung aus dem nicht-metallischen Material eine Klebstoffschicht auf.

Die Erfindung sieht vor, dass die Beschichtungen aus einem nichtmetallischen Material zwischen der jeweiligen Flächenseite des Grundkörpers und der Klebstoffschicht angeordnet sind. In diesen Fällen dienen die Klebstoffschichten als Haftvermittler zwischen der Oberfläche der Beschichtungen aus dem nicht-metallischen Material und anderen Oberflächen. Die Klebstoffschicht sollte die Flächenseite des Grundkörpers, auf die sie aufgebracht ist, vollständig abdecken.

Sind mehrere Klebstoffschichten vorgesehen, so können diese aus derselben oder unterschiedlichen Klebemassen gebildet sein. Bevorzugt sind sämtliche Klebstoffschichten aus derselben Klebemasse gebildet.

Die Stärke einer Klebstoffschicht liegt im Bereich von 10 nm bis 800 µm, besonders bevorzugt 1 µm bis 500 µm und am stärksten bevorzugt 20 µm bis 200 µm. Handelt es sich bei dem Grundkörper um einen folienförmigen Grundkörper (beispielsweise eine Bleifolie), so liegt die Stärke der Klebstoffschicht stärker bevorzugt zwischen 10 nm und 500 nm, besonders bevorzugt zwischen 50 nm und 250 nm.

Eine besonders bevorzugte Ausgestaltung der Erfindung sieht vor, dass die Stärke der Klebstoffschicht in einem Bereich zwischen 80 und 120 µm liegt. Es hat sich herausgestellt, dass diese Schichtdicke für eine besonders gute Haftung sorgt.

In einer bevorzugten Ausführungsform ist die erste Flächenseite des Grundkörpers mit einer Beschichtung aus dem nicht-metallischen Material versehen. Die zweite Flächenseite des Grundkörpers ist mit einer Klebstoffschicht beschichtet. Diese Klebstoffschicht ist nicht von einer Beschichtung aus einem nicht-metallischen Material bedeckt. Sie ist daher zur Herstellung einer Klebeverbindung zwischen dem Strahlenschutzformkörper und einer Oberfläche, die mit einem solchen Strahlenschutzformkörper verkleidet werden soll, geeignet.

Der Grundkörper besteht aus Blei oder einer Bleilegierung. Blei ist aufgrund seiner leichten Verformbarkeit und seines niedrigen Schmelzpunktes eines der am längsten verwendeten Metalle. Die Zugfestigkeit des Bleis sollte im Bereich von 13 bis 20 N/mm² liegen. Die Zugfestigkeit einer Bleilegierung sollte im Bereich von 13 bis 40 N/mm² liegen. Blei und Bleilegierungen sind resistent gegen Salz- und Schwefelsäuren und wirken abschirmend gegen alpha-, beta- und gamma-Strahlung. Blei hat eine Dichte von 11,34 g/cm³. Es ist beliebig oft recyclebar.

Vorzugsweise ist der Grundkörper ein folienförmiger Grundkörper. Ein folienförmiger Grundkörper wird im Folgenden auch als Bleifolie bezeichnet.

Vorzugsweise ist jede der Oberflächen, d. h. die Flächenseiten und Schmalseiten, des Grundkörpers, mit einer Beschichtung versehen, so dass der Grundkörper vollflächig umhüllt ist.

In der vorliegenden Erfindung wird unter einem nicht-metallischen Material jedes Material verstanden, dass kein metallisches Material ist. Vorzugsweise ist das nicht-metallische Material ein organisches Material, stärker bevorzugt ein polymeres Material. Besonderes bevorzugt ist das nicht-metallische Material aus der Gruppe ausgewählt, die Polymere wie Polyethylen, Polypropylen, Polyvinylchlorid, Polyethylenterephthalat und Silikone umfasst. Das nicht-metallische Material sollte durch nicht-ionische Strahlung nicht oder nur gering beeinträchtigt werden. Es sollte ferner UV-beständig sein.

Das nicht-metallische Material sollte eine Bearbeitungstemperatur im Bereich von -10 bis 80 °C aufweisen. Es sollte den Sprühnebeltest über 1008 h nach ECCA T8 ohne Befund überstehen. Eine Schnellbewitterung über 1008 h nach QUV-UVB 313 Kreidung sollte einen Wert von weniger als 10 %Ganzgrad 2E ergeben. Eine Wasserlagerung über 1008 h sollte ohne Befund sein. Die Kälteverformbarkeit über 75 h bei -10 °C nach QMH-4.10-QW 1156 sollte ohne Befund sein.

Die Klebstoffschicht ist ebenfalls eine Schicht aus einem nicht-metallischen Material. Sie weist jedoch zusätzlich dazu klebende Eigenschaften auf. Die Klebstoffschicht ist eine Klebemasse auf Acrylat-Basis, besonders bevorzugt eine Poly(meth)acrylat-Klebemasse, beispielsweise eine modifizierte Acrylat-Klebemasse.

Die Klebstoffschicht sollte einerseits über die nötige Klebkraft zur Oberfläche des Grundkörpers als auch über eine hohe Klebkraft auf seiner

Außenseite verfügen, so dass der Strahlenschutzformkörper mittels der Klebstoffschicht auf einer Wandfläche befestigt werden kann. Die Klebkraft sollte durch nicht-ionische Strahlung nicht oder nur gering beeinträchtigt werden. Nach einer Strahlenbelastung von 1,5 MGy über 24 h vermindert sich die Klebkraft vorzugsweise um maximal 40 %. Ferner sollte die Klebkraft an der Außenseite der Klebstoffschicht auch bei punktuellen Höchstbelastungen oder Dauerbestrahlungen noch eine ausreichende Höhe besitzen.

Der Klebstoff wird in Abhängigkeit von dem späteren Verwendungszweck des Strahlenschutzformkörpers ausgewählt. Die Klebemasse auf Acrylat-Basis haftet beispielweise auf Metall, Kunststoff, Holz, Papier, Gipskarton, Mauerwerk, Putz, Beton und beschichteten Oberflächen. Die Klebstoffe werden üblicherweise als Rollen- oder Planware bereitgestellt, so dass Klebstoffe vergleichweise einfach auf die Oberflächen des Grundkörpers laminiert werden können.

Jede Klebstoffschicht kann aus Lagen unterschiedlicher Klebstoffe aufgebaut sein, wodurch ein mehrschichtiger, sandwichartiger Aufbau der Klebstoffschicht erhalten wird.

Die Verarbeitungstemperatur der Klebemasse sollte im Bereich von 10 bis 25 °C liegen. Die Anwendungstemperatur sollte im Bereich von -40 bis 100 °C, kurzzeitig bis zu 200 °C liegen.

Vorzugsweise liegt die Abzugskraft der Klebemasse von der Oberfläche des Grundkörpers (gemessen gemäß Afera 5001) bei 23,1 N/25mm, bei einem sandwichartigen Aufbau der Klebeschicht bei 20,1 N/25 mm.

Die Klebemasse kann Partikel aus Blei oder einer Bleilegierung aufweisen, um die Abschirmungseigenschaften des erfindungsgemäßen Strahlenschutzformkörpers weiter zu verbessern. Die Partikel sollten dabei homogen in der Klebstoffschicht verteilt sein. Die Partikel haben vorzugsweise eine Teilchengröße im Nano- oder Mikrometerbereich, bevorzugt 10 bis 500 nm, stärker bevorzugt 10 bis 100 nm.

Zumindest eines der nicht-metallischen Materialien und/oder die Klebstoffschicht können bakterizid und/oder schmutzabweisend ausgerüstet sein. Auf diese Weise wird die Haltbarkeit der nicht-metallischen Beschichtung

Der erfindungsgemäße Strahlenschutzformkörper ist besonders zur Verwendung als Abschirmung gegen ionisierende Strahlung, insbesondere künstliche ionisierende Strahlung geeignet. Ist der Grundkörper als Bleifolie ausgebildet, so kann der erfindungsgemäße Strahlenschutzformkörper aufgrund der Flexibilität der Bleifolie in Art einer Tapete zur Innenauskleidung von Räumen verwendet werden, was insbesondere im medizinischen Bereich von Vorteil ist. Dabei ist vorzugsweise die erste Flächenseite der Bleifolie mit einer Beschichtung aus einem organischen, bevorzugt polymeren Material versehen, während die andere, zweite Flächenseite der Bleifolie mit einer ersten Klebstoffschicht versehen ist. Es wird also der Strahlenschutzformkörper als Strahlenschutzfolie erhalten, die auf einfache Weise über die erste Klebstoffschicht an Wänden befestigt werden kann. Dabei ist die Beschichtung aus dem nicht-metallischen Material der Wand abgewandt angeordnet. Die Eigenschaften dieser Beschichtung ermöglichen es, einen Raum mit einer hygienischen, abriebfesten, verschleißbeständigen, schmutzabweisenden und UVbeständigen Verkleidung zu versehen.

### Kurze Beschreibung der Zeichnungen

Die Erfindung wird nachfolgend anhand von Beispielen, die die Erfindung nicht beschränken sollen, unter Bezugnahme auf die Zeichnungen näher erläutert. Dabei zeigen:
Fig. 1 eine schematische Schnittdarstellung eines ersten Beispiels eines Strahlenschutzformkörpers;
Fig. 2 eine schematische Schnittdarstellung eines zweiten Beispiels eines Strahlenschutzformkörpers;
Fig. 3 eine schematische Schnittdarstellung eines dritten Beispiels eines Strahlenschutzformkörpers;
Fig. 4 eine schematische Schnittdarstellung eines vierten Beispiels eines Strahlenschutzformkörpers;
Fig. 5 eine schematische Schnittdarstellung eines fünften Beispiels, das einer Ausführungsform des erfindungsgemäßen Strahlenschutzformkörpers entspricht;
Fig. 6 eine schematische Perspektivdarstellung eines sechsten Beispiels eines Strahlenschutzformkörpers mit abschnittsweise entfernter Beschichtung; und
Fig. 7 eine schematische Schnittdarstellung des in Fig. 6 gezeigten

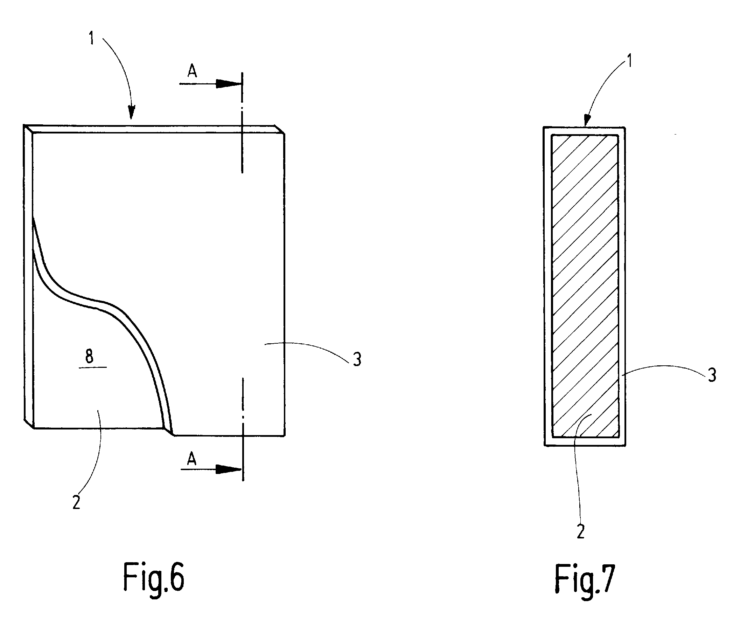

Beispiels entlang Schnittlinie A-A in Fig. 6.

### Beispiele der Offenbarung

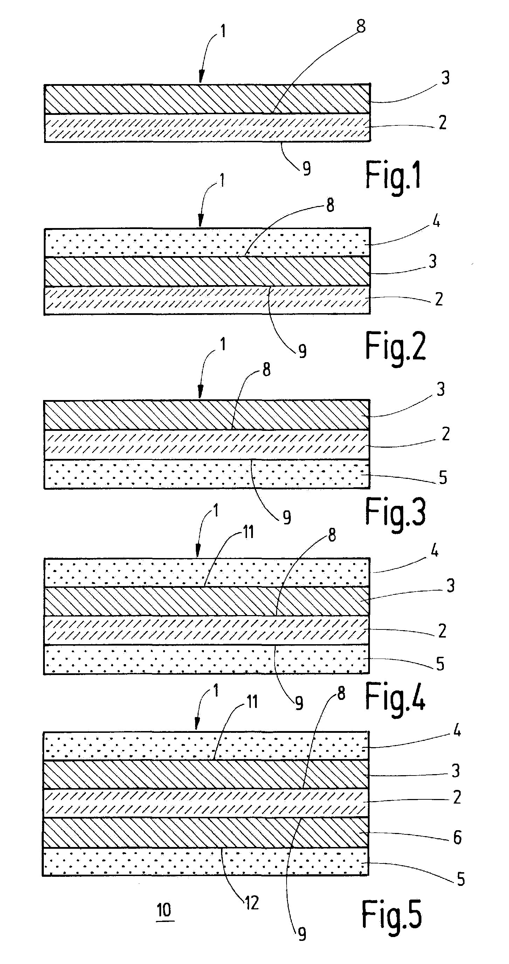

Die in Fig. 1 gezeigte Schnittdarstellung zeigt einen Querschnitt durch ein erstes Beispiel eines folienförmigen Strahlenschutzformkörpers 1. Der Grundkörper des Strahlenschutzformkörpers 1 ist eine Bleifolie 2. Die erste, obere Flächenseite 8 der Bleifolie 2 ist vollständig mit einer Beschichtung 3 aus einem nicht-metallischen Material versehen. Dazu wird das nicht-metallische Material auf die erste Flächenseite der Bleifolie 2, beispielsweise mittels eines Extruders, unter Ausbildung der Beschichtung 3 laminiert. Die zweite, untere Flächenseite 9 der Bleifolie 2 ist nicht beschichtet. Auch die Schmalseiten der Bleifolie 2 sind nicht beschichtet, was aufgrund der geringen Stärke der Bleifolie 2 nicht erforderlich ist. Eine Beschichtung der Schmalseiten mit einer Beschichtung aus dem nicht-metallischen Material kann jedoch vorgesehen sein.

Die in Fig. 2 gezeigte Schnittdarstellung zeigt einen Querschnitt durch ein zweites Beispiel eines folienförmigen Strahlenschutzformkörpers 1. Der Grundkörper des Strahlenschutzformkörpers 1 ist eine Bleifolie 2. Die erste, obere Flächenseite 8 der Bleifolie 2 ist vollständig mit einer ersten Beschichtung 3 aus einem nicht-metallischen Material abgedeckt. Auf der oberen Flächenseite 8 der Beschichtung 3 ist eine Klebstoffschicht 4 aufgebracht, die die obere Flächenseite 8 der Beschichtung 3 vollständig abdeckt. Dazu wird zunächst das nicht-metallische Material unter Ausbildung der Beschichtung 3 auf die erste Flächenseite 8 der Bleifolie 2 und anschließend die Klebstoffmasse unter Ausbildung der Klebstoffschicht 4 auf die erste Beschichtung 3 aus dem nichtmetallischen Material jeweils beispielsweise mittels eines Extruders, laminiert. Die zweite, untere Flächenseite 9 der Bleifolie 2 ist nicht beschichtet. Auch die Schmalseiten der Bleifolie 2 sind nicht beschichtet, was aufgrund der geringen Stärke der Bleifolie 2 nicht erforderlich ist. Eine Beschichtung der Schmalseiten der Bleifolie 2 mit einer Beschichtung aus dem nicht-metallischen Material kann jedoch vorgesehen sein.

Die in Fig. 3 gezeigte Schnittdarstellung zeigt einen Querschnitt durch ein drittes Beispiel eines folienförmigen Strahlenschutzformkörpers 1. Der Grundkörper des Strahlenschutzformkörpers 1 ist eine Bleifolie 2. Auf die erste, obere Flächenseite 8 der Bleifolie 2 ist eine Beschichtung 3 aus einem nicht-metallischen Material aufgebracht, die die obere Flächenseite der Bleifolie vollständig abdeckt. Auf die zweite, untere Flächenseite 9 der Bleifolie 2 ist eine Klebstoffschicht 5 aufgebracht, die die untere Flächenseite der Bleifolie 2 vollständig abdeckt. Dazu wird entweder zunächst die Klebstoffmasse unter Ausbildung der Klebstoffschicht 5 auf die zweite Flächenseite der Bleifolie 2 und anschließend das nicht-metallische Material unter Ausbildung der Beschichtung 3 auf die erste Flächenseite der Bleifolie, jeweils beispielsweise mittels eines Extruders, laminiert oder umgekehrt. Auch eine gleichzeitige Laminierung beider Flächenseiten der Bleifolie 2 ist möglich. Die Schmalseiten der Bleifolie 2 sind nicht beschichtet, was aufgrund der geringen Stärke der Bleifolie 2 nicht erforderlich ist. Die Klebstoffschicht 5 ermöglicht ein Aufkleben des Strahlenschutzformkörpers auf eine Wand. Eine Beschichtung der Schmalseiten mit einer Beschichtung aus dem nicht-metallischen Material kann jedoch vorgesehen sein, so dass der Strahlenschutzformkörper 1 vollflächig von nicht-metallischem Material und Klebstoff umhüllt ist.

Die in Fig. 4 gezeigte Schnittdarstellung zeigt einen Querschnitt durch ein viertes Beispiel eines folienförmigen Strahlenschutzformkörpers 1. Der Grundkörper des Strahlenschutzformkörpers 1 ist eine Bleifolie 2. Die erste, obere Flächenseite 8 der Bleifolie 2 ist vollständig mit der ersten Beschichtung 3 aus einem nicht metallischen Material abgedeckt. Auf der dritten, oberen Flächenseite 11 der Beschichtung 3 ist eine Klebstoffschicht 4 aufgebracht, die die obere Flächenseite, d. h. die der Bleifolie 2 abgewandte Seite der Beschichtung 3 vollständig abdeckt. Dazu wird zunächst das nicht-metallische Material unter Ausbildung der Beschichtung 3 auf die erste Flächenseite 8 der Bleifolie 2 und anschließend die Klebstoffmasse unter Ausbildung der Klebstoffschicht 4 auf die Beschichtung 3, jeweils beispielsweise mittels eines Extruders, laminiert. Auf die zweite, untere Flächenseite 9 der Bleifolie 2 ist eine Klebstoffschicht 5 aufgebracht, die die untere Flächenseite 9 der Bleifolie 2 vollständig abdeckt. Das Laminieren der Klebstoffmasse 5 auf die untere Flächenseite der Bleifolie 2 kann vor, nach oder gleichzeitig mit der Laminierung der oberen Flächenseite 8 erfolgen. Die Klebstoffschicht 5 ermöglicht ein Aufkleben des Strahlenschutzformkörpers auf eine in Fig. 5 bei 10 angedeutete Wand, wohingegen die Klebstoffschicht 4 als Haftfläche beispielsweise für Gegenstände und Vorrichtungen dienen kann. Die Schmalseiten der Bleifolie 2 sind nicht beschichtet, was aufgrund der geringen Stärke der Bleifolie 2 nicht erforderlich ist. Eine Beschichtung der Schmalseiten mit einer Beschichtung aus dem nicht-metallischen Material kann jedoch vorgesehen sein, so dass der Strahlenschutzformkörper 1 vollflächig von nicht-metallischem Material und Klebstoff umhüllt ist.

Die in Fig. 5 gezeigte Schnittdarstellung zeigt einen Querschnitt durch ein fünftes Beispiel eines erfindungsgemäßen folienförmigen Strahlenschutzformkörpers 1.

Der Grundkörper des Strahlenschutzformkörpers 1 ist eine Bleifolie 2. Die erste, obere Flächenseite 8 der Bleifolie 2 ist vollständig mit einer ersten Beschichtung 3 aus nicht metallischem Material abgedeckt. Auf der oberen Flächenseite 11 der Beschichtung 3 ist eine Klebstoffschicht 4 aufgebracht, die die obere Flächenseite 11 der Beschichtung 3 vollständig abdeckt. Dazu wird zunächst das nicht-metallische Material unter Ausbildung der Beschichtung 3 auf die erste Flächenseite 8 der Bleifolie 2 und anschließend die Klebstoffmasse unter Ausbildung der Klebstoffschicht 4 auf die Beschichtung 3, jeweils beispielsweise mittels eines Extruders, laminiert. Die zweite, untere Flächenseite 9 der Bleifolie 2 ist vollständig mit einer Beschichtung 6 aus einem nicht metallischen Material abgedeckt. Auf die vierte, untere Flächenseite 12 der Beschichtung 6 aus einem nicht-metallischen Material ist eine Klebstoffschicht 5 aufgebracht, die die untere Flächenseite 12 der Beschichtung 6 vollständig abdeckt. Dazu wird zunächst das nicht-metallische Material unter Ausbildung der Beschichtung 6 auf die untere Flächenseite 12 der Bleifolie 2 und anschließend die Klebstoffmasse unter Ausbildung der Klebstoffschicht 5 auf die untere Flächenseite 12 der Beschichtung 6, jeweils beispielsweise mittels eines Extruders, laminiert. Das Laminieren der unteren Flächenseite 9 der Bleifolie 2 kann vor, nach oder gleichzeitig mit der Laminierung der oberen Flächenseite 8 der Bleifolie 2 erfolgen. Die Schmalseiten der Bleifolie sind nicht beschichtet, was aufgrund der geringen Stärke der Bleifolie 2 nicht erforderlich ist. Eine Beschichtung der Schmalseiten mit einer Beschichtung aus dem nicht-metallischen Material kann jedoch vorgesehen sein, so dass der Strahlenschutzformkörper 1 vollflächig von nicht-metallischem Material und zusätzlich, insbesondere vollflächig, auch von Klebstoff umhüllt ist.

Die in den Fig. 6 und 7 gezeigte sechstes Beispiel des Strahlenschutzformkörpers 1 zeigt einen plattenförmigen Grundkörper 2. Sowohl die Flächenseiten 8, 9 des Grundkörpers 2 als auch die Schmalseiten des Grundkörpers 2 sind mit Beschichtungen 3 aus einem nicht-metallischen Material abgedeckt, so dass der Grundkörper 2 vollständig von dem nicht-metallischen Material umhüllt ist. Selbstverständlich kann ein Strahlenschutzformkörper mit plattenförmigem Grundkörper auch den in den Fig. 1 bis 5 gezeigten Aufbau haben, wobei anstelle der Bleifolie eine Bleiplatte eingesetzt wird.

### Bezugszeichenliste

- 1: Strahlenschutzformkörper
- 2: Grundkörper
- 3: erste Beschichtung aus nicht-metallischem Material
- 4: erste Klebstoffschicht
- 5: zweite Klebstoffschicht
- 6: zweite Beschichtung aus nicht-metallischem Material
- 8: erste Flächenseite
- 9: zweite Flächenseite
- 10: Wand
- 11: dritte Flächenseite
- 12: vierte Flächenseite

## Patentansprüche

1. Strahlenschutzformkörper (1), im Querschnitt umfassend einen Grundkörper (2) aus Blei oder einer Bleilegierung, wobei der Grundkörper (2) ein plattenförmiger, blechförmiger oder folienförmiger Grundkörper (2) mit quadratischem, rechteckförmigem oder eine andere geometrische Form aufweisendem Zuschnitt ist und eine erste Flächenseite (8), die mit einer ersten Beschichtung (3) versehen ist, die aus einem ersten nicht-metallischen Material besteht, und eine zweite Flächenseite (9) aufweist, die mit einer zweiten Beschichtung (6) versehen ist, wobei die Beschichtung (6) aus einem zweiten nicht-metallischen Material besteht, wobei das erste nicht-metallische Material und das zweite nicht-metallische Material gleich oder unterschiedlich sind, und wobei eine obere Flächenseite (11) der ersten Beschichtung (3) mit einer Klebstoffschicht (4) und eine untere Flächenseite (12) der zweiten Beschichtung (6) mit einer Klebstoffschicht (5) versehen ist, wobei jede Beschichtung (3, 6) aus einem nicht-metallischen Material zwischen der jeweiligen Flächenseite (8, 9) des Grundkörpers (2) und der Klebstoffschicht (4, 5) angeordnet ist, und die Beschichtung (3, 6) aus dem nicht-metallischen Material eine Stärke im Bereich von 10 nm bis 100 µm aufweist, und wobei die Klebstoffschicht (4, 5) eine Klebemasse auf Acrylat-basis ist und eine Stärke im Bereich von 10 nm bis 800 µm aufweist.

2. Strahlenschutzformkörper nach Anspruch 1, **wobei** der Grundkörper (2) eine biegefähige Bleifolie ist.

3. Strahlenschutzformkörper nach einem der vorstehenden Ansprüche 1 oder 2, **wobei** der Grundkörper (2) vollflächig von dem ersten nicht-metallischen Material umhüllt ist.

4. Strahlenschutzformkörper nach einem der vorstehenden Ansprüche, **wobei** der Grundkörper (2) vollflächig von dem zweiten nicht-metallischen Material umhüllt ist.

5. Strahlenschutzformkörper nach einem der vorstehenden Ansprüche, **wobei** das erste nicht-metallische Material aus der Gruppe ausgewählt ist, die Polymere wie Polyethylen, Polypropylen, Polyvinylchlorid, Polyethylenterephthalat und Silikone umfasst.

6. Strahlenschutzformkörper nach einem der vorstehenden Ansprüche, **wobei** das zweite nicht-metallische Material aus der Gruppe ausgewählt ist, die Polymere wie Polyethylen, Polypropylen, Polyvinylchlorid, Polyethylenterephthalat und Silikone umfasst.

7. Strahlenschutzformkörper nach einem der vorstehenden Ansprüche, **wobei** die Beschichtung (3, 6) aus dem nicht-metallischen Material eine Stärke im Bereich von 10 bis 30 µm aufweist.

8. Strahlenschutzformkörper nach einem der vorstehenden Ansprüche, **wobei** die Klebstoffschicht (4, 5) eine Klebemasse auf Acrylatbasis mit einer Stärke im Bereich von 20 µm bis 200 µm ist.

9. Strahlenschutzformkörper nach einem der vorstehenden Ansprüche, **wobei** die Klebstoffschicht (4, 5) eine Stärke im Bereich von 80 bis 120 µm aufweist.

10. Strahlenschutzformkörper nach einem der Ansprüche 2 bis 9, **wobei** die Beschichtungen (3, 6) auf Basis modifizierter Acrylatharzdispersionen hergestellt sind.

11. Verwendung des Strahlenschutzkörpers nach einem der Ansprüche 1 bis 10 zur Abschirmung gegen die Einwirkung gegen ionisierende Strahlung.

12. Verwendung nach Anspruch 11, **wobei** der Strahlenschutzformkörper (1) einen plattenförmigen, blechförmigen oder folienförmigen Grundkörper (2) aufweist, wobei die erste Flächenseite (8) des Grundkörpers (2) mit einem nicht-metallischen Material beschichtet ist und wobei die zweite Flächenseite (9) des plattenförmigen, blechförmigen oder folienförmigen Grundkörpers (2) mit einer Klebstoffschicht (5) beschichtet ist.

13. Verwendung nach Anspruch 12, **wobei** der Strahlschutzformkörper (1) als Tapete verwendet wird, wobei die die Klebstoffschicht (5) aufweisende Flächenseite der Tapete einer, insbesondere verkleideten, Wand (10) zugewandt ist.

## Claims

1. A molded radiation protection element (1), in cross section comprising a base body (2) of lead or lead alloy, wherein the base body (2) is a plate-shaped, metal plate-shaped or film-shaped base body (2) with a cut having a square, rectangular or a different geometric, and has a first surface side (8) which is provided with a first coating (3) which consists of a first non-metallic material and a second surface side (9) that is provided with a second coating (6), wherein the coating (6) consists of a second non-metallic material, wherein the first non-metallic material and the second non-metallic material are the same or different, and wherein an upper surface side (11) of the first coating (3) is provided with an adhesive layer (4) and a lower surface side (12) of the second coating (6) is provided with an adhesive layer (5), wherein each coating (3, 6) made of a non-metallic material is arranged between the respective surface side (8,9) of the base body (2) and the adhesive layer (4, 5), and the coating (3, 6) of the non-metallic material has a thickness in the range of 10 nm to 100 µm, and wherein the adhesive layer (4, 5) is an adhesive compound on acrylate basis and has a thickness in the range of 10 nm to 800 µm

2. A molded radiation protection element according to claim 1, **wherein** the base body (2) is a bendable lead film.

3. A molded radiation protection element according to one of the preceding claims 1 or 2, **wherein** the base body (2) is wrapped by the first non-metallic material across its entire surface.

4. A molded radiation protection element according to one of the preceding claims, **wherein** the base body (2) is wrapped by the second non-metallic material across its entire surface.

5. A molded radiation protection element according to one of the preceding claims, **wherein** the first non-metallic material is chosen from the group comprising polymers such as polyethylene, polypropylene, polyvinylchloride, polyethylene terephthalate and silicones.

6. A molded radiation protection element according to one of the preceding claims, **wherein** the second non-metallic material is chosen from the group comprising polymers such as polyethylene, polypropylene, polyvinylchloride, polyethylene terephthalate and silicones.

7. A molded radiation protection element according to one of the preceding claims, **wherein** the coating (3, 6) of the non-metallic material has a thickness in the range of 10 to 30 µm.

8. A molded radiation protection element according to one of the preceding claims, **wherein** the adhesive layer (4, 5) is an adhesive compound based on acrylate with a thickness in the range of 20 µm to 200 µm.

9. A molded radiation protection element according to one of the preceding claims, **wherein** the adhesive layer (4, 5) has a thickness in the range of 80 µm to 120 µm.

10. A molded radiation protection element according to one of the preceding claims, **wherein** the coating (3, 6) are produced on the basis of modified acrylate resin dispersions.

11. A use of the molded radiation protection element according to one of the claims 1 to 10 for shielding against the impact of ionizing radiation.

12. A use according to claim 11, **wherein** the molded radiation protection element (1) has a plate-shaped, metal plate-shaped or film-shaped base body (2), wherein the first surface side (8) of the base body (2) is coated with a non-metallic material and wherein the second surface side (9) of the plate-shaped, metal plate-shaped or film-shaped basic body (2) is coated with an adhesive layer (5).

13. A use according to claim 12, **wherein** the molded radiation protection element (1) is used as wallpaper, wherein the surface side of the wallpaper having an adhesive layer (5) faces a, in particular, lined wall (10).

## Revendications

1. Corps moulé de protection contre les radiations (1), comprenant en section transversale un corps de base (2) en plomb ou en un alliage de plomb, dans lequel le corps de base (2) est un corps de base (2) en forme de plaque, en forme de tôle ou en forme de feuille avec une pièce carrée, rectangulaire ou présentant une autre forme géométrique et présente une première face plate (8), qui est munie d'un premier revêtement (3) qui se compose d'un premier matériau non métallique, et une deuxième face plate (9), qui est munie d'un deuxième revêtement (6), dans lequel le revêtement (6) se compose d'un deuxième matériau non métallique, dans lequel le premier matériau non métallique et le deuxième matériau non métallique sont identiques ou différents, et dans lequel une face plate supérieure (11) du premier revêtement (3) est munie d'une couche de colle (4) et une face plate inférieure (12) du deuxième revêtement (6) est munie d'une couche de colle (5), dans lequel chaque revêtement (3, 6) en un matériau non métallique est disposé entre la face plate respective (8, 9) du corps de base (2) et la couche de colle (4, 5), et le revêtement (3, 6) en ce matériau non métallique présente une épaisseur comprise dans la plage de 10 nm à 100 µm, et dans lequel la couche de colle (4, 5) est une pâte adhésive à base acrylate et présente une épaisseur comprise dans la plage de 10 nm à 800 µm.

2. Corps moulé de protection contre les radiations selon la revendication 1, dans lequel le corps de base (2) est une feuille de plomb flexible.

3. Corps moulé de protection contre les radiations selon une des revendications 1 ou 2, dans lequel le corps de base (2) est enveloppé sur toute la surface par le premier matériau non métallique.

4. Corps moulé de protection contre les radiations selon l'une quelconque des revendications précédentes, dans lequel le corps de base (2) est enveloppé sur toute la surface par le deuxième matériau non métallique.

5. Corps moulé de protection contre les radiations selon l'une quelconque des revendications précédentes, dans lequel le premier matériau non métallique est choisi dans le groupe qui comprend des polymères comme le polyéthylène, le polypropylène, le chlorure de polyvinyle, le téréphtalate de polyéthylène et les silicones.

6. Corps moulé de protection contre les radiations selon l'une quelconque des revendications précédentes, dans lequel le deuxième matériau non métallique est choisi dans le groupe, qui comprend des polymères comme le polyéthylène, le polypropylène, le chlorure de polyvinyle, le téréphtalate de polyéthylène et les silicones.

7. Corps moulé de protection contre les radiations selon l'une quelconque des revendications précédentes, dans lequel le revêtement (3, 6) en ce matériau non métallique présente une épaisseur comprise dans la plage de 10 à 30 µm.

8. Corps moulé de protection contre les radiations selon l'une quelconque des revendications précédentes, dans lequel la couche de colle (4, 5) est une pâte adhésive à base acrylate avec une épaisseur comprise dans la plage de 20 µm à 200 µm.

9. Corps moulé de protection contre les radiations selon l'une quelconque des revendications précédentes, dans lequel la couche de colle (4, 5) présente une épaisseur comprise dans la plage de 80 à 120 µm.

10. Corps moulé de protection contre les radiations selon l'une quelconque des revendications 2 à 9, dans lequel les revêtements (3, 6) sont fabriqués à base de dispersions de résine acrylate modifiées.

11. Utilisation du corps moulé de protection contre les radiations selon l'une quelconque des revendications 1 à 10 pour le blindage contre l'action d'un rayonnement ionisant.

12. Utilisation selon la revendication 11, dans laquelle le corps moulé de protection contre les radiations (1) présente un corps de base (2) en forme de plaque, en forme de tôle ou en forme de feuille, dans laquelle la première face plate (8) du corps de base (2) est revêtue avec un matériau non métallique et dans laquelle la deuxième face plate (9) du corps de base (2) en forme de plaque, en forme de tôle ou en forme de feuille est revêtue avec une couche de colle (5).

13. Utilisation selon la revendication 12, dans laquelle le corps moulé de protection contre les radiations (1) est utilisé comme tapisserie, dans laquelle la face plate de la tapisserie présentant la couche de colle (5) est tournée vers une paroi (10), en particulier recouverte.
